# EUROPEAN PATENT APPLICATION

(11) **EP 1 614 746 A1**
(43) Date of publication of application: **11.01.2006**
(21) Application number: 04721362.4
(22) Date of filing: 17.03.2004
(51) Int. Cl.: C12N 9/06, C12N 15/53, C12N 15/10, C12N 1/15, C12N 1/19, C12N 1/21, C12Q 1/26, C12M 1/40, G01N 27/30, G01N 27/48

(54) **FRUCTOSYLAMINE OXIDASE**

(30) Priority: 17.03.2003 JP 2003116348
(71) Applicant: Sode, Koji, Tokyo 152-0013 (JP)
(72) Inventor: Sode, Koji, Tokyo 152-0013 (JP)
(74) Representative: Vossius & Partner
(86) International application number: PCT/JP2004/003587
(87) International publication number: WO 2004/083419

(57) **Abstract**

A novel fructosylamine oxidase derived from yeast Pichia sp. and a gene coding for the enzyme are disclosed. The fructosylamine oxidase may be produced by culturing the transformant which has been transformed with a recombinant vector comprising the DNA fragment containing the fructosylamine oxidase gene. Since the fructosylamine oxidase oxidizes fructosyl valine, it may be used in an assay for fructosyl valine. The assay kit and enzyme sensor comprising the fructosylamine oxidase of the invention are useful in measurement of glycated hemoglobin.

SEQUENCE LISTING

<110>  Sode, Koji

<120>  Fructosylamine Oxidase

<140>  PCT/JP2004/003587

<150>  JP 2003-116348

<151>  2003-03-17

<160>  15

<170>  PatentIn version 3.1

<210>  1

<211>  427

<212>  PRT

<213>  Pichia sp.

<400>  1

Met Glu Ser Ile Ile Ile Val Gly Ala Gly Thr Phe Gly Leu Ser Thr
1               5                   10                  15

Ala Leu Gln Leu Ala Arg Asp Gly Tyr Lys Asn Ile Lys Cys Phe Asp
            20                  25                  30

Lys Phe Pro Val Pro Ser Glu Ile Ala Ala Gly Asn Asp Ser Asn Lys
        35                  40                  45

Ile Phe His Tyr Asp Tyr Val Ala Pro Leu Ala Lys Pro Asn Ser Lys
    50                  55                  60

Glu Arg Leu Ser Leu Glu Ala Leu His Leu Trp Lys Thr Asp Pro Val
65                  70                  75                  80

Tyr Lys Pro Tyr Tyr His Pro Val Gly Phe Ile Leu Ala Ala Ser Ser
                85                  90                  95

Asp Ala Pro Leu Leu His Asp Lys Glu Tyr Tyr Glu Glu Leu Gln Lys
            100                 105                 110

Asn Gly Leu Arg Asn Tyr Arg Tyr Ile Ser Thr Pro Glu Glu Phe Arg
        115                 120                 125

Glu Tyr Leu Pro Ile Leu Lys Gly Pro Leu Pro Asn Trp Arg Gly Tyr

130                 135                 140
Val Leu Asp Gly Asp Asn Gly Trp Leu His Ala Arg Asp Ser Leu Lys
145                 150                 155                 160
Ser Ala Tyr Glu Glu Cys Lys Arg Leu Gly Val Glu Phe Val Phe Gly
                165                 170                 175
Asp Asp Gly Glu Ile Val Glu Leu Leu Asn Glu Asn Gly Lys Leu Thr
            180                 185                 190
Gly Ile Arg Ala Arg Ser Gly Ala Ile Phe Ser Ala Gln Lys Tyr Val
        195                 200                 205
Leu Ser Ser Gly Ala Asn Ala Val Thr Leu Leu Asn Phe Gln Arg Gln
    210                 215                 220
Leu Glu Gly Lys Cys Phe Thr Leu Ala His Phe Lys Val Thr Asp Glu
225                 230                 235                 240
Glu Ala Lys Ala Phe Lys Ser Leu Pro Val Leu Phe Asn Ala Glu Lys
                245                 250                 255
Gly Phe Phe Phe Glu Ala Asp Glu Asn Asn Glu Ile Lys Ile Cys Asn
            260                 265                 270
Glu Tyr Pro Gly Phe Thr His Thr Asn Glu Ser Gly Glu Ser Ile Pro
        275                 280                 285
Leu Tyr Arg Met Glu Ile Pro Leu Glu Ser Ala Leu Glu Ile Arg Gln
    290                 295                 300
Tyr Leu Lys Glu Thr Met Pro Gln Phe Ala Asp Arg Pro Phe Thr Lys
305                 310                 315                 320
Thr Arg Ile Cys Trp Cys Thr Asp Ser Pro Asp Met Gln Leu Ile Leu
                325                 330                 335
Cys Thr His Pro Glu Tyr Thr Asn Leu Ile Val Ala Ser Gly Asp Ser
            340                 345                 350
Gly Asn Ser Phe Lys Ile Met Pro Ile Ile Gly Lys Tyr Val Ser Lys
        355                 360                 365
Val Val Thr Lys Gly Asp Lys Gly Leu Asp Pro Glu Asp Lys Glu Cys

370                 375                 380

Trp Lys Trp Arg Pro Glu Thr Trp Asp Lys Arg Gly Gln Val Arg Trp

385                 390                 395                 400

Gly Gly Arg Tyr Arg Val Ala Asp Leu Asn Glu Ile Glu Glu Trp Val

                405                 410                 415

Ser Val Glu Asn Pro Thr Pro His Lys Leu Glu

            420                 425

<210> 2

<211> 1284

<212> DNA

<213> Pichia sp.

<400> 2

atggagtcga taattatagt tggtgccggt acttttgggc tttccacagc cttacagctt      60

gccagagatg gatacaagaa cataaaatgt tttgacaagt ttccggttcc atctgagata     120

gctgctggaa acgacagtaa caagattttt cactacgatt atgttgctcc cctggctaaa     180

cccaattcaa aagaacggtt gagtctcgaa gcattacacc tttggaagac agatccggtg     240

tacaaaccgt actatcatcc ggtaggattt atcctggctg caagttccga tgctccatta     300

ctgcatgata aggaatacta tgaagagttg caaaaaaacg gacttcgcaa ttatcgttat     360

atttcaactc ccgaggagtt tcgtgagtat ttgcccattt taaagggccc gttacccaac     420

tggagaggat atgttctcga cggagataac ggatggttgc atgctcgaga ctcattgaaa     480

agtgcatacg aagaatgcaa acgattggga gtggaatttg tgtttggaga cgatggggaa     540

attgtcgaat tacttaacga aaatggaaag ttgacgggaa ttagggccag atctggtgcc     600

atattctcgg cacaaaaata tgttctcagc tctggtgcaa atgcagtaac gttgttaaat     660

ttccagagac agctagaagg taaatgtttc actttggcac atttcaaagt gacggatgaa     720

gaagctaaag catttaaaag cttgccggtc cttttcaatg ccgaaaaagg gttttttttc     780

gaggctgatg aaaataacga aatcaaaatt tgcaacgagt accctggatt tacccacaca     840

aatgaatccg gagagtctat cccactctac cggatggaga ttccactcga gtcagcactt     900

gaaattagac aatacttgaa agaaaccatg cctcagtttg ctgatagacc tttcaccaag     960

acaagaattt gttggtgtac cgactctccc gacatgcaat tgatcttgtg tactcaccca    1020

gaatacacca accttattgt agcatcgggt gactctggaa attcgttcaa gatcatgcca    1080

atcattggca aatatgtcag caaggttgtt accaaaggtg ataaaggatt ggatccggaa 1140
gataaagaat gctggaaatg gcgtcctgag acttgggaca agcgggggca ggtccgctgg 1200
ggtggtcgat accgtgttgc ggatttgaac gaaattgaag aatgggtttc tgttgaaaat 1260
cccacaccac acaaactaga ataa 1284
<210> 3
<211> 13
<212> PRT
<213> Pichia sp.
<400> 3
Gly Phe Phe Phe Glu Ala Asp Glu Asn Asn Glu Ile Lys
1               5                   10
<210> 4
<211> 17
<212> PRT
<213> Pichia sp.
<400> 4
Phe His Tyr Asp Tyr Val Ala Pro Leu Ala Lys Pro Asn Ser Lys Glu
1               5                   10                  15
Arg
<210> 5
<211> 27
<212> PRT
<213> Pichia sp.
<400> 5
Asp Ala Pro Leu Leu His Asp Lys Glu Tyr Tyr Glu Glu Leu Gln Lys
1               5                   10                  15
Asn Gly Leu Arg Asn Tyr Arg Tyr Ile Ser Thr
            20                  25
<210> 6

<211> 12

<212> PRT

<213> Pichia sp.

<400> 6

Thr Lys Gly Asp Lys Gly Leu Asp Pro Glu Asp Lys

1               5                   10

<210> 7

<211> 13

<212> PRT

<213> Pichia sp.

<400> 7

Trp Val Ser Val Glu Asn Pro Thr Pro His Lys Leu Glu

1               5                   10

<210> 8

<211> 29

<212> DNA

<213> Artificial Sequence

<220>

<223> PCR primer

<220>

<221> variation

<222> 3

<223> n is a, g, c or t

<220>

<221> variation

<222> 6

<223> n is a, g, c or t

<220>

<221> variation

<222> 12

<223> n is a, g, c or t
<220>
<221> variation
<222> 15
<223> n is a, g, c or t
<220>
<221> variation
<222> 18
<223> n is a, g, c or t
<220>
<221> variation
<222> 21
<223> n is a, g, c or t
<220>
<221> variation
<222> 24
<223> n is a, g, c or t
<220>
<221> variation
<222> 27
<223> n is a, g, c or t
<400> 8
ggnacntggg gnwsnwsnac ngcnytnca 29
<210> 9
<211> 26
<212> DNA
<213> Artificial Sequence
<220>
<223> PCR primer
<220>

<221> variation
<222> 9
<223> n is a, g, c or t
<220>
<221> variation
<222> 24
<223> n is a, g, c or t
<400> 9
tcytcrtyng gytcvawraa raancc 26
<210> 10
<211> 30
<212> DNA
<213> Artificial Sequence
<220>
<223> PCR primer
<400> 10
atttcaaagt gacggatgaa gaagctaaag 30
<210> 11
<211> 21
<212> DNA
<213> Artificial Sequence
<220>
<223> PCR primer
<400> 11
cgcagttttc ccagtcacga c 21
<210> 12
<211> 33
<212> DNA
<213> Artificial Sequence
<220>

<223>  PCR primer

<400>  12

gtgcatacga agaatgcaaa cgattgggag tgg                                    33

<210>  13

<211>  32

<212>  DNA

<213>  Artificial Sequence

<220>

<223>  PCR primer

<400>  13

ccatccgtta tctccgtcga gaacatatcc tc                                     32

<210>  14

<211>  29

<212>  DNA

<213>  Artificial Sequence

<220>

<223>  PCR primer

<400>  14

atcaccatgg agtcgataat tatagttgg                                         29

<210>  15

<211>  30

<212>  DNA

<213>  Artificial Sequence

<220>

<223>  PCR primer

<400>  15

ttgattctag acatgtatgt tgtaatcttg                                        30

## Description

### Technical Field

The present invention relates to a novel fructosylamine oxidase (FAO). More particularly, the present invention relates to a fructosylamine oxidase produced by a novel microorganism and to a process for producing the same. The present invention also relates to a gene coding for fructosylamine, a recombinant vector having incorporated a gene fragment coding for the FAO, a transformant transformed by the recombinant vector and a process for producing FAO by culturing the transformant. The present invention further relates to a kit and a sensor for the measurement of glycated albumin, fructosamine, HbA1c and fructosyl valine using fructosylamine oxidase produced according to the present invention.

### Background Art

Amino groups on the backbone and the side chain of a protein binds non-enzymatically to the reducing terminal of a reducing sugar such as glucose to give an Amadori compound, a glycated compound. It has been known that hemoglobin is glycated in blood to generate glycated hemoglobin (glycohemoglobin; HbA1c). Since the abundance ratio of HbA1c to hemoglobin is higher in patients suffering from diabetes mellitus as compared with a normal healthy individual and the concentration of HbA1c in blood reflects the blood-sugar level during several weeks in the past, concentration of HbA1c in blood is quite important in clinical tests for diagnosis of diabetes mellitus and as an index for blood-sugar control of patients suffering from diabetes mellitus.

In HbA1c, glucose is bound to valine at the N-terminal of the β-chain of hemoglobin and, therefore, fructosyl valine is able to be used as a low-molecular model compound for HbA1c. Thus, it is possible to measure HbA1c using an enzyme having specificity to fructosyl valine.

Until now, enzymes acting on Amadori compound have been isolated from various kinds of strains and it has been suggested that glycated protein such as glycated albumin, HbA1c and fructosamine may be analyzed using such enzymes (Japanese Patent Laid-Open No. 61/268,178; Japanese Patent Laid-Open No. 61/280,297; Japanese Patent Laid-Open No. 03/155,780; Japanese Patent Laid-Open No. 05/192,193; Japanese Patent Laid-Open No. 07/289,253; Japanese Patent Laid-Open No. 08/154,672; *Agric. Biol. Chem.,* 53(1), 103-110, 1989; *Agric. Biol. Chem.,* 55(2), 333-338, 1991; *J. Biol. Chem.,* 269(44), 27297-27301, 1994; *Appl. Environ. Microbiol.,* 61(12), 4487-4489, 1995; *Biosci. Biotech. Biochem.,* 59(3), 487-491, 1995; *J. Biol. Chem.,* 270(1), 218-224, 1995; *J. Biol. Chem.,* 271(51), 32803-32809, 1996; and *J. Biol. Chem*., 272(6), 3437-3443, 1997).

An object of the present invention is to clone the structural gene of a novel fructosylamine oxidase which is able to react with fructosyl valine, to clarify an amino acid sequence thereof, as well as to provide a process for production of the enzyme using a recombinant DNA technique and an analytical method using the enzyme based on the information obtained in the present invention.

### Disclosure of the Invention

The present inventors have conducted various investigations and succeeded in cloning a gene coding for novel fructosylamine oxidase (FAO) to accomplish the present invention. It has also been found that FAO is able to be produced in large quantities by culturing a transformant prepared by transformation of a microorganism with a recombinant vector incorporated with a DNA fragment containing a gene coding for fructosylamine oxidase (FAO), and collecting FAO from the culture.

Thus, the present invention provides a process for production of FAO, comprising culturing a transformant prepared by transformation of a microorganism with a recombinant vector incorporated with a DNA fragment containing a gene coding for FAO, and collecting FAO from the culture.

The present invention also provides a protein having a fructosylamine oxidase activity of the following (a) or (b).
(a) a protein comprising the amino acid sequence as set forth in SEQ ID NO: 1 of the Sequence Listing;
(b) a protein comprising an amino acid sequence where one or several amino acid residue(s) is/are deleted, substituted or added in the amino acid sequence of (a) and having a fructosylamine oxidase activity.

The present invention also provides a gene coding for a fructosylamine oxidase which is a protein of the following (a) or (b):
(a) a protein comprising the amino acid sequence as set forth in SEQ ID NO: 1 of the Sequence Listing;
(b) a protein comprising an amino acid sequence where one or several amino acid residue(s) is/are deleted, substituted or added in the amino acid sequence of (a) and having a fructosylamine oxidase activity.
   The present invention also provides a gene coding for a fructosylamine oxidase and having a sequence of the following (c) or (d):
(c) DNA comprising the nucleotide sequence as set forth in SEQ ID NO: 2 of the Sequence Listing;
(d) DNA where one or several nucleotide (s) is /are deleted, substituted or added in the above sequence (c) and codes for a protein having a fructosylamine oxidase activity.

The present invention also provides a fructosylamine oxidase comprising the sequence:

GlyPhePhePheGluAlaAspGluAsnAsnGluLleLys.

The present invention provides a fructosylamine oxidase having any of the sequences of the following (e) to (h):

(e)

PheHisTyrAspTyrValAlaProLeuAlaLysProAsnSerLysGluArg;

(f)

AspAlaProLeuLeuHisAspLysGluTyrTyrGluGluLeuGlnLysAsnGlyLeuA

rgAsnTyrArgTyrIleSerThr;

(g) ThrLysGlyAspLysGlyLeuAspProGluAspLys; and

(h) TrpValSerValGluAsnProThrProHisLysLeuGlu.

The present invention also provides a recombinant vector containing a gene coding for the fructosylamine oxidase of the present invention and a transformant or a transfectant transformed with the recombinant vector.

The present invention also provides a process for production of a fructosylamine oxidase comprising culturing the transformant of the present invention and collecting the fructosylamine oxidase from the culture, as well as a fructosylamine oxide produced by such a process.

In another aspect, the present invention provides an analytical method for a fructosylamine compound, such as fructosamine, glycated albumin, fructosyl valine and HbA1c using the fructosylamine oxidase of the present invention. The analysis may be conducted either spectroscopically or electrochemically. In a preferred embodiment, the present invention provides a method for assaying fructosamine, glycated albumin or HbA1c, comprising digesting fructosamine, glycated albumin or HbA1c in a sample to provide a fructosylamine compound such as fructosyl valine, and analyzing the fructosylamine compound spectroscopically using the fructosylamine oxidase of the present invention. The present invention also provides a fructosyl valine assay kit and an HbA1c assay kit containing the fructosylamine oxidase of the present invention.

In another aspect, the present invention provides an enzyme electrode having the fructosylamine oxidase of the present invention immobilized thereon, and an enzyme sensor comprising the enzyme electrode as a working electrode.

### Brief Description of Drawings

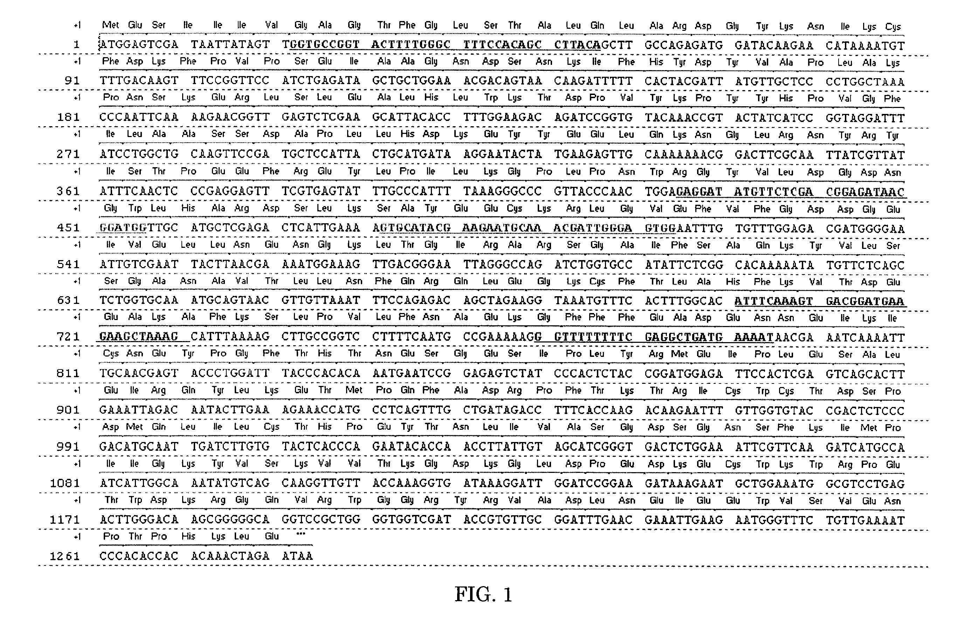

Fig. 1 shows the amino acid sequence and the nucleotide sequence of a fructosylamine oxidase derived from *Pichia* sp. N1-1 strain. The amino acid sequence corresponds to SEQ ID NO: 1 of the Sequence Listing and the nucleotide sequence corresponds to SEQ ID NO: 2 of the Sequence Listing.

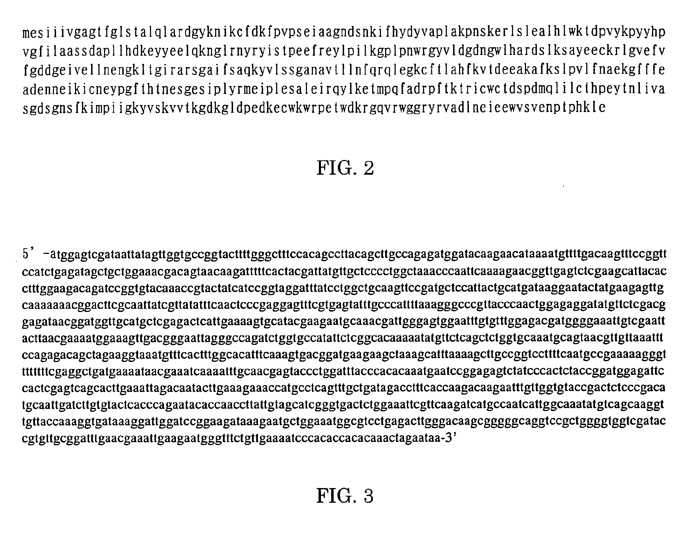

Fig. 2 shows SEQ ID NO: 1 of the Sequence Listing showing the amino acid sequence of a fructosylamine oxidase derived from *Pichia* sp. N1-1 strain.
Fig. 3 shows SEQ ID NO: 2 of the Sequence Listing showing the nucleotide sequence of a fructosylamine oxidase derived from *Pichia* sp. N1-1 strain.

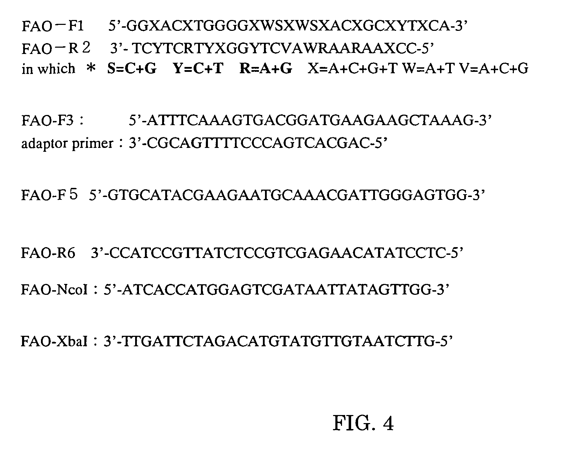

Fig. 4 shows PCR primers used in the present invention.

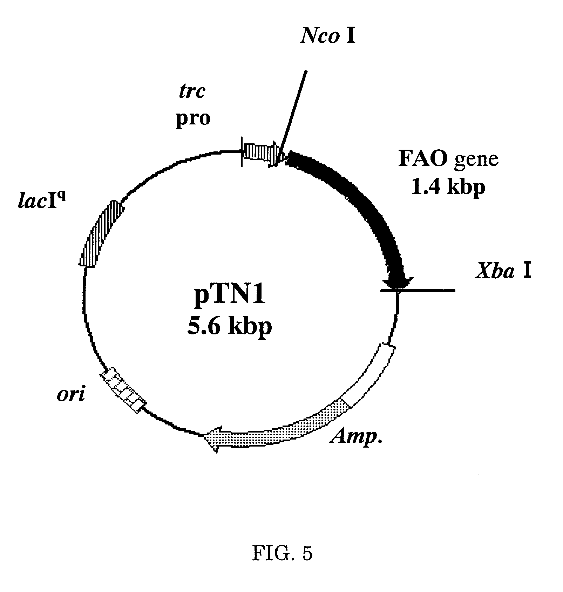

Fig. 5 shows an expression vector pTN1.
Fig. 6 shows production of a recombinant FAO in *Escherichia coli.*
Fig. 7 shows an electrophoretic picture of a recombinant FAO.
Fig. 8 shows the activity and substrate specificity of a recombinant FAO.

### Detailed Description of the Invention

### Fructosylamine oxidase

Until now, there has been no report for structural gene of fructosylamine oxidase from yeast and genus *Pichia.* Accordingly, structural gene and amino acid sequence of the enzyme of the invention are believed to be novel, and a process for producing the same using a recombinant DNA technique is a novel process.

### Method for isolation of gene

A DNA fragment containing the gene coding for FAO of the present invention may be isolated from FAO-producing microorganisms. Various strains of genus Pichia, for example, may be used as a FAO-producing microorganism, and among them *Pichia pastoris* is most suitable.

Other examples of the FAO-producing microorganism containing a gene coding for FAO of the present invention include various yeast strains, such as *Pichia acaciae, Pichia alni, Pichia ambrosiae, Pichia Americana*, *Pichia amylophila, Pichia angophorae, Pichia angusta, Pichia anomala, Pichia barkeri, Pichia besseyi, Pichia bimundalis, Pichia bispora, Pichia bovis, Pichia buntonii, Pichia cactophila, Pichia canadensis, Pichia capsulate, Pichia castillae, Pichia chambardii, Pichia ciferrii, Pichia delftensis, Pichia deserticola, Pichia dianae, Pichia dorogensis, Pichia dryadoides, Pichia etchellsii, Pichia euphorbiae, Pichia euphorbiiphila, Pichia fabianii, Pichia farinose, Pichia fermentans, Pichia finlandica, Pichia fluxuum, Pichia galeiformis, Pichia glucozyma, Pichia gluiliermondii, Pichia hampshirensis, Pichia haplophila, Pichia hawaiiensis, Pichia heedii, Pichia heimii, Pichia henricii, Pichia holstii, Pichia inositovora, Pichia jadinii (Torulla yeast), Pichia japonica, Pichia kluyveri, Pichia kodamae, Pichia lachancei, Pichia lynferdii, Pichia maclurae, Pichia manshurica, Pichia media, Pichia membranifaciens, Pichia methanolica, Pichia methylivora, Pichia mexicana, Pichia meyerae, Pichia minuta, Pichia mississippiensis, Pichia misumaiensis, Pichia naganishii, Pichia nakasei, Pichia nakazawae, Pichia norvegensis, Pichia ofunaensis, Pichia ohmeri, Pichia onychis, Pichia petersonii, Pichia philodendra, Pichia philogaea, Pichia pijperi, Pichia pilisensis, Pichia pinus, Pichia populi, Pichia pseudocactophila, Pichia pseudopastoris, Pichia quercuum, Pichia rabaulensis, Pichia ramenticola, Pichia rhodanensis, Pichia salicis, Pichia salictaria, Pichia scolyti, Pichia segobiensis, Pichia silvicola, Pichia spartinae, Pichia sporocuriosa, Pichia stipitis, Pichia strasburgensis, Pichia subpelliculosa, Pichia sydowiorum, Pichia tannicola, Pichia toletana, Pichia trehaloabstinens, Pichia trehalophila, Pichia triangularis, Pichia veronae, Pichia wickerhamni, Pichia xylose* and *Pichia szoltii.*

The gene coding for the FAO may be extracted from those strains or may be chemically synthesized. It is also possible to prepare a DNA fragment containing FAO gene utilizing a PCR method.

Examples of the above-mentioned gene coding for the FAO are (a) a gene coding for a protein comprising the amino acid sequence as set forth in SEQ ID NO: 1 of the Sequence Listing and (b) a gene coding for a protein comprising an amino acid sequence where one or several amino acid residue (s) in the amino acid sequence (a) is/are deleted, substituted or added and having FAO activity.

Further examples include (c) DNA comprising the nucleotide sequence as set forth in SEQ ID NO: 2 of the Sequence Listing and (d) DNA where one or several nucleotide(s) is/are deleted, substituted or added in the above-mentioned sequence (c) and coding for FAO.

The gene coding for FAO of the present invention may be obtained by the following methods. For example, chromosome is separated and purified and DNA is cleaved by means of ultrasonic treatment, restriction enzyme treatment, to prepare a fragment, which is then ligated with a linearized expression vector by DNA ligase at blunt end or sticky end of both DNAs to construct a recombinant vector. After the recombinant vector is transfected to a replicable host microorganism, the transfectant is screened based on the marker on the vector and the expression level of the enzymatic activity to select a microorganism carrying the recombinant vector containing the gene coding for FAO.

Next, the above microorganism carrying the recombinant vector is cultured, and the recombinant vector is separated and purified from the cells of the cultured microorganism. The gene coding for FAO is isolated from the expression vector. For example, chromosomal DNA which is a gene donor may be isolated as follows.

The gene donor microorganism is cultured with shaking for 1 to 3 day(s), and the culture medium is centrifuged to collect the cells, which are lysed to prepare lysate containing the FAO gene. The cells may be lysed by treatment with a lytic enzyme such as lysozyme. If necessary, protease or other enzyme and surfactant such as sodium laurylsulfate (SDS) may be used together. It is also possible to combine with a physical disruption method such as freeze-thaw and French press treatment.

DNA may be separated and purified from the lysate prepared as above using any of common methods such as removal of protein by treating with phenol or with protease, treatment with ribonuclease or precipitating with alcohol and combination thereof.

DNA separated and purified from microorganisms may be cleaved by ultrasonic treatment or restriction enzyme treatment. Preferably, a type-II restriction enzyme acting on a specific nucleotide sequence is conveniently used.

A vector used for cloning includes various constructs for genetic recombination which are derived from a plasmid or phage capable of autonomously growing in a host microorganism. Examples of the phage include Lambda gt10 and Lambda gt11 when *Escherichia coli* is used as a host microorganism. Examples of the plasmid include pBR322, pUC18, pUC118 , pUC19, pUC119 , pTrc99A, pBluescript and pET28 and a cosmid SuperCosI when *E. coli* is used as a host microorganism.

With regard to a host microorganism used for the cloning, any type of microorganism may be used as long as it can stably maintain the recombinant vector, grow autonomously, and allow for expression of exogenous genes. Generally, *Escherichia coli* DH5α, XL-1 Blue MR, *Escherichia coli* BL21, etc. may be used. Since the enzyme of the invention is derived from yeast *Pichia,* it is also possible to use eukaryotic microorganisms such as *Saccharomyces cerevisiae* or *Pichia pastorius* as a host.

A method for transfection of recombinant vector into the host microorganism includes an electroporation method or a competent cell method by a calcium treatment when a host microorganism is *Escherichia coli.*

The resulted transformant is able to produce a large amount of FAO in a stable manner when it is cultured in a nutrient medium. The transformant carrying the recombinant vector of interest may be selected based on the expression of the drug-resistant marker on the vector and the FAO activity. For example, a microorganism may be selected which grows in a selective medium corresponding to the drug-resistant marker and produces FAO.

In another aspect of the invention, the amino acid sequence may be determined from the isolated and purified FAO, and based on the sequence information FAO structural gene is cloned from genomic DNA or cDNA prepared from the strain.

For example, pPreparation of cDNA from N1-1 strain may be conducted as follows. First, mRNA is extracted from the N1-1 strain. After the N1-1 strain is pre-cultured in YM medium (at 30°C, 5 ml, L-shaped tube), it is inoculated in an amount of 1% to M9 minimum medium (M9/FV) (initial concentration: 0.01; 30°C; 100 ml, 500 ml baffle) supplemented with fructosyl valine or FV (final concentration: 0.48%) as a sole nitrogen source. The cells are collected (5,000 gm, 15 minutes, 4°C) at about OD₆₆₀ = 3, and resuspended in 200 µl of buffer A (1M sorbitol, 100 mM EDTA and 14 mM mercaptoethanol). A zymolyase solution (1,000 U zymolyase/1 ml buffer A) (20 µl) is added and mixed, then allowed to stand at 30°C for 30 minutes. After centrifugation (15,000 rpm, 10 minutes, 4°C), the precipitate is suspended in 1 mL of Isogen and allowed to stand at room temperature for 5 minutes. To this 0.2 ml of chloroform is added and mixed, and allowed to stand at room temperature for 3 minutes. After centrifugation (15,000 rpm, 15 minutes, 4°C), 0.5 ml of isopropanol is added to the aqueous phase and mixed, and allowed to stand at room temperature for 10 minutes. After centrifugation (15,000 rpm, 10 minutes, 4°C), 1 ml of 70% ethanol is added to the precipitate and mixed. After centrifugation (15,000 rpm, 5 minutes, 4°C), the precipitate is dried *in vacuo* for 15 minutes. The mRNA thus prepared may be used for the synthesis of cDNA.

A fragment containing FAO structural gene may be amplified by a common method for the preparation of cDNA from mRNA by an RT-PCR. First, mRNA is subjected to a reverse transcription reaction using an oligo dT adapter primer according to the manual of an RNA-PCR kit (AMV Ver. 2.1, Takara). After cDNA is purified by ethanol precipitation , PCR is carried out using it as a template. Examples of the PCR primers include FAO-F1 which is designed from a conserved sequence of FAODs reported, and FAO-R2 which is designed from 13 amino acid residues obtained from amino acid sequence analysis of FAOD derived from the N1-1 strain.
FAO-F1 5'-GGXACXTGGGGXWSXWSXACXGCXYTXCA-3'
FAO-R2

3'-TCYTCRTYXGGGYTCVAWRAARAAXCC-5'

in which S = C + G, Y = C + T, R = A + G, X = A + C + G + T, W = A + T, V = A + C + G.

PCR amplification may be carried out under the following reaction conditions:
one cycle of:
   at 94°C for 1 minute,
   at 60°C for 30 seconds and
   at 72°C for 30 seconds; and
25 cycles of:
   at 98°C for 30 seconds,
   at 60°C for 30 seconds,
   at 72°C for 7 minutes and
   at 72°C for 8 minutes.

As Taq polymerase, it is possible to use TaKaRa LA *Taq*™ or the like. PCR product is subjected to a glass milk purification according to the manual attached to Gene Clean II Kit, then subcloned into a vector according to the manual attached to pGEM-T Vector System. A transformant carrying the vector having an inert is selected by color selection. The transformant is cultured in LB medium (37°C, 1.8 ml, test tube), then the cells are collected and the plasmid is extracted. The plasmid is analyzed for the nucleotide sequence of the insert using M13 Primer Forward and M13 Primer Reverse (Takara Bio). Preparation and analysis of a sample may be carried out in accordance with, for example, the manual attached to ABI Prism 310 Genetic Analyzer (Perkin-Elmer Applied Biosystem).

Then, PCR is carried out to analyze the nucleotide sequence of the C-terminal region. PCR is conducted using the purified cDNA as a template. Examples of the PCR primers include FAO-F3 designed from the sequence obtained from analysis of the nucleotide sequence of the insert and an adapter primer sequence.
FAO-F3 5'-ATTTCAAAGTGACGGATGAAGAAGCTAAAG-3'
Adapter primer:

3'-CGCAGTTTTCCCAGTCACGAC-5'

On the basis of the internal partial sequence information of FAO determined from cDNA prepared as above, the full length structural gene of this enzyme may be cloned by inverse PCR using genomic DNA of N1-1 strain as a template.

For example, genomic DNA from the N1-1 strain may be extracted as follows. After N1-1 strain is cultured in YM medium (30°C, 100 ml, 300 ml baffle), 25 ml of ethanol and 1 ml of 0. 5M EDTA are added to 25 ml of culture medium and allowed to stand at -30°C for 30 minutes. The cells are collected (5,000 G, 15 minutes, 4°C) and resuspended in 1 ml of aseptic ultrapure water. After centrifugation (8,000 rpm, 5 minutes, 4°C), the cells are resuspended in 0.5 ml of spheroplast buffer (1.2 M sorbitol, 0.1 M EDTA, 1% mercaptoethanol and 0.1% zymolyase) and allowed to stand at 37°C for 30 minutes. To this is added 0.5 ml of proteinase K buffer (50 mM EDTA, 0.3% SDS, 0.01% proteinase K) and the mixture is allowed to stand at 65°C for 30 minutes. To this is added 0.2 ml of 5M potassium acetate and the mixture is allowed to stand on ice for 10 minutes. After centrifugation (10,000 rpm, 15 minutes, 4°C), the supernatant liquid is precipitated with ethanol and the precipitate is dried *in vacuo* for 15 minutes. The precipitate is dissolved in 500 µl of TE buffer (10 mM NaCl, 20 mM Tris-HCl (pH 8.0), 1 mM EDTA), 5 µl of RNase is added and the mixture is allowed to stand at 37°C for 30 minutes. Then it is extracted with phenol-chloroform and precipitated with ethanol. The precipitate is dried *in vacuo* and then dissolved in 1 ml of aseptic ultrapure water.

The genomic DNA derived from N1-1 strain may be used for amplification of FAOD structural gene fragment by inverse PCR. The genomic DNA is digested with a restriction enzyme and allowed for self-ligation according to the manual attached to DNA Ligation Kit Ver. 2. PCR is carried out using the resulting cyclic DNA as a template. Primers FAO-F5 and FAO-R6 designed from the partial sequence of the structural gene of FO are used as primers.
FAO-F5 5'-GTGCATACGAAGAATGCAAACGATTGGGAGTGG-3'
FAO-R6

3'-CCATCCGTTATCTCCGTCGAGAACATATCCTC-5'

PCR amplification is carried out under the following reaction conditions:
one cycle of:
   at 94°C for 1 minute,
   at 60°C for 30 seconds and
   at 72°C for 30 seconds; and
25 cycles of:
   at 98°C for 30 seconds,
   at 60°C for 30 seconds,
   at 72°C for 7 minutes and
   at 72°c for 8 minutes.

As Taq polymerase, it is possible to use TaKaRa LA *Taq*™ or the like. The PCR product is purified again and the nucleotide sequence is analyzed according to a common method.

The target gene may be obtained by analyzing the nucleotide sequence of the PCR amplified fragment. On the basis of the sequence information of FAO gene, a gene containing the full-length fragment may be prepared by PCR amplification from N1-1 genomic DNA. In order to amplify the sequence containing such gene regions, the following primers are designed.
FAO-NcoI: 5'-ATCACCATGGAGTCGATAATTATAGTTGG-3'
FAO-XbaI:

3'-TTGATTCTAGACATGTATGTTGTAATCTTG-5'

Using these primers, genomic DNA containing the fragment of interest may be amplified by PCR.

The reaction is carried out according to the following cycles:
at 94°C for 5 minutes
(at 94°C for 30 seconds
at 55°C for 30 seconds
at 72°C for 1 minute) x 25 cycles
at 72°C for 5 minutes.

The nucleotide sequence of FAO gene obtained by the above-mentioned method may be confirmed by a fully automated nucleotide sequence analyzer by a common method. It is also possible to deduce the amino acid sequence of FAO from the nucleotide sequence determined as above.

FAO gene may be transferred from the vector obtained as above to a recombinant vector capable of replicating in a microorganism. For example, FAO gene is recovered from the vector with a restriction enzyme or PCR and then ligated to another vector fragment. Transformation of a microorganism with such a vector may be carried our by electroporation or a competent cell method by treatment with calcium.

FAO derived from N1-1 strain may be recombinantly produced in *Escherichia coli.* Primers complementary to the N-terminal and C-terminal are designed from the structural gene sequence information of FAOD derived from N1-1 strain. In designing the primers, *Nco* I recognition sequence (FAO-Nco I) is added to a primer at the N-terminal side while *Xba* I recognition sequence (FAO-Xba I) is added to a primer at the C-terminal.
FAO-NcoI: 5'-ATCACCATGGAGTCGATAATTATAGTTGG-3'
FAO-XbaI:

3'-TTGATTCTAGACATGTATGTTGTAATCTTG-5'

FAOD structural gene is amplified by PCR using the above-mentioned primers using genomic DNA or cDNA is used as a template. The PCR product is digested with *Nco* I and *Xba* I and ligated (DNA Ligation Kit, Ver. 2) with a high-expression vector pTrc99a (Invitrogen) digested with the same restriction enzymes to provide a plasmid (pTN1). A recombinant FAOD may be produced in *Escherichia coli* transformed with the plasmid pNT1.

### Production of recombinant FAOD

The transformant may be cultured under conditions selected in terms of the nutritive and physiological property of the host. In many cases, they are conveniently cultured in a liquid medium. In an industrial scale, it is advantageous to culture the transformant with aeration and stirring.

For example, *Escherichia coli* DH5α is transformed with pTN1 and cultured in LB medium (30°C, 150 ml, baffle, 50 µg/ml ampicillin). IPTG (final concentration: 0.3 mM) is added when OD₆₀₀ reaches about 0.7. Then the culture is continued at a lower temperature of 30°C and 2 ml aliquot is collected every one hour. The cells are disrupted with ultrasonication, and FAOD activity of the supernatant is measured to calculate the production per liter (U/L culture). The activity is determined by measuring the absorbance at 505 nm according to the POD/phenol/4A.A. method using FV (2 mM), PPb (pH 7.0, 50 mM), phenol (1.5 mM), 4-aminoantipyrine (1.5 mM) and peroxidase (2U/ml).

Purification of the recombinant FAO may be carried out as follows. First, a water-soluble fraction containing the enzyme is prepared. Recombinant *Escherichia coli* pTN1/DH5α is cultured in LB medium (37°C, 7 1, 10 1 fermenter, 50 µg/ml ampicillin), then induced with IPTG (final concentration: 0.3 mM) at about OD₆₆₀ = 0.7 and culture temperature is lowered to 30°C. The cells are suspended in 100 mM PPb (pH 7.0) and disrupted four times using a French press. A supernatant liquid is ultracentrifuged (40,000 g, 90 minutes) and the supernatant is dialyzed against 10 mM PPb (pH 7.0) at 4°C overnight to prepare a water-soluble fraction. The water-soluble fraction may be subjected to liquid chromatography as follows to obtain a purified enzyme.

The enzyme is purified with an anion-exchange chromatography (DEAE-5PW). The water-soluble fraction is adsorbed to an anion-exchange chromatography column DEAE-5PW (5.0 mm I.D. x 5 cm, manufactured by Tosoh) equilibrated with 10 mM PPb (pH 7.0). After it is equilibrated with 10 mM PPb (pH 7.0) in a 3-fold of the column volume, FAOD is eluted with 10 mM PPb (pH 7.0) containing 0.7 M of NaCl. The flow rate is set to 1 ml/min and eluate is collected every one minute. The eluate is monitored by the absorbance at 280 nm. The active fraction is separated with 35% ammonium sulfate and the supernatant liquid is subjected to a hydrophobic chromatography. The active fraction is adsorbed to a hydrophobic chromatography column (Resource Phe; 1 ml, Pharmacia) equilibrated with 10 mM PPb (pH 6.5) containing 35% ammonium sulfate. After equilibration with 10 mM PPb (pH 6.5) containing 35% ammonium sulfate in a 3-fold of the column volume, FAOD is eluted with 10 mM PPb (pH 6.5). The flow rate is set to 2 ml/min and eluate is collected every one minute. The active fraction is separated with 45% ammonium sulfate, then the precipitate is dissolved in 10 mM PPb (pH 7.0) containing 1% mannose and 100 µM FAD and dialyzed against the same buffer at 4°C for 6 hours. It is further dialyzed against 10 mM PPb (pH 8.0) containing 100 µM FAD at 4°C for 6 hours. The dialyzed sample is subjected to the next anion-exchange chromatography. The sample is adsorbed to an anion-exchange chromatography column (Bioasit Q; 4.6 mm I. D. × 5 cm, Tosoh) equilibrated with 10 mM PPb (pH 8.0). After equilibration with 10 mM PPb (pH 8.0) in a 3-fold of the column volume, FAOD is eluted with 10 mM PPb (pH 7.0) containing 0.3 M NaCl. The flow rate is set to 1 ml/min and eluate is collected every one minute. The active fraction is dialyzed against 10 mM PPb (pH 7.0) at 4°C overnight. Degree of purification of the sample thus prepared may be examined by SDS/PAGE. The sample is subjected to electrophoresis using Phast Gel 8-25 and the gel is stained with silver. Sample preparation, electrophoresis and staining may be conducted in accordance with the manual attached to Phast System™.

The purified enzyme may be dried to powder by means of, for example, freeze-drying, vacuum drying or spray drying to obtain a product.

### Fructosylamine assay kit and sensor

A kit for the measurement of fructosyl valine may be constructed using the enzyme of the present invention. In addition to the fructosylamine oxidase of the invention, the kit contains buffer necessary for the measurement, appropriate mediator and, if necessary, enzymes such as peroxidase, standard solution of fructosyl valine or a derivative thereof for the preparation of a calibration curve and an instruction for use. The fructosylamine oxidase of the present invention may be provided in various forms, for example, as a freeze-dried reagent or as a solution in an appropriate storage solution.

It is also possible to construct fructosamine, glycated albumin or HbA1c assay kit using the enzyme of the present invention. Fructosylamine, glycated albumin or HbA1c is enzymatically or chemically digested to generate a fructosylamine compound such as fructosyl valine, which in turn is quantified using the fructosylamine oxidase of the present invention. In this way, fructosamine, glycated albumin or HbA1c may be assayed. Accordingly, the assay kit of the present invention for fructosamine, glycated albumin or HbA1c may further contain a reagent for hydrolysis or protease in the above-mentioned kit for measurement of fructosyl valine.

It is also possible to construct a sensor for the measurement of fructosyl valine and a sensor for the measurement of fructosamine, glycated albumin or HbA1c using the fructosylamine oxidase of the present invention. The concentration of the substrate, i.e., a fructosylamine compound such as fructosyl valine, may be determined by measuring consumed oxygen or generated hydrogen peroxide by the action of the fructosylamine oxidase of the present invention. Various sensor systems for the measurement of oxygen or hydrogen peroxide have been known in the art. Oxygen electrode, carbon electrode, metal electrode, platinum electrode, etc. are used as electrodes and the enzyme of the present invention is immobilized on the electrodes. Examples of the means for immobilization include cross-linking, encapsulating into a macromolecular matrix, coating with a dialysis membrane, optical cross-linking polymer, electroconductive polymer, oxidation-reduction polymer, and any combination thereof.

When an oxygen electrode is used, the enzyme of the present invention is immobilized on the electrode surface, and the electrode is inserted into a buffer and kept at a predetermined temperature. A sample is added and a decreased value of electric current is measured. When measurement is conducted in an amperometric system using carbon electrode, gold electrode or platinum electrode provided with an immobilized enzyme is used as a working electrode, together with a counter electrode (such as platinum electrode) and a reference electrode (such as Ag/AgCl electrode). The electrodes are inserted into a buffer containing a mediator and kept at predetermined temperature. Predetermined voltage is applied to the working electrode, then a sample is added and increased value in electric current is measured. Examples of the mediator used in the assay include potassium ferricyanide, ferrocene, osmium derivative, ruthenium derivative, phenazine methosulfate, etc.

Further, fructosylamine may be assayed using a immobilized electron mediator in an amperometric system using carbon electrode, gold electrode, or platinum electrode. The enzyme is immobilized on the electrode together with an electron mediator such as potassium ferricyanide, ferrocene, osmium derivative, phenazine methosulfate in a macromolecular matrix by means of adsorption or covalent bond to prepare a working electrode. It is inserted into buffer together with a counter electrode (such as platinum electrode) and a reference electrode (such as Ag/AgCl electrode), and kept at a predetermined temperature. Predetermined voltage is applied to the working electrode, then the sample is added and increased value in electric current is measured.

To prepare a sensor for the measurement of fructosylamine, glycated albumin or HbA1c, the above-mentioned sensor for the measurement of fructosyl valine is further combined with a membrane containing immobilized proteinase (such as protease) to construct a complex sensor. The structure of such a complex sensor based on a continuous reaction by a combination of plural enzymes is well known in the art. See, for example, "Biosensor - Fundamental and Applications" by Anthony P. F. Tuner, Isao Karube and George S. Wilson, Oxford University Press, 1987.

Contents of all patents and reference documents explicitly cited in the present specification are entirely incorporated herein by reference. Further, disclosure of the specification and the drawings of Japanese Patent Application No. 2003/116,348 which is a base for claiming a priority of the present application are entirely incorporated herein by reference.

### Examples

The present invention will be illustrated in detail by way of the Examples below, although the present invention shall not be limited to those Examples.

### Example 1

### Determination of internal amino acid sequence of FAO

The strain was incubated according to the publication "Screening and Characterization of Fructosyl-valine utilizing Marine Microorganisms" by K. Sode, et al., *Mar. Biotechnol.,* 3, 126-132 (2001). Purified enzyme of FAO derived from *Pichia* sp. N1-1 was obtained. A sample of the purified enzyme was freeze-dried and ultrapure water was added to 145 µg of the sample to the protein concentration of 2 mg/ml. The sample was subjected to SDS-PAGE (10% polyacrylamide gel) using a Lapidus-Slab electrophoretic device. The gel of the SDS-PAGE was cut out in a size of 5 × 5 cm and blotted to a PVDF membrane for 2 hours using Phast System™. The PVDF membrane was stained with Coomassie Blue and the target band was cut out. The enzyme was digested with trypsin and separated by a reversed phase liquid chromatography. One of the fractions of the analyzed pattern was used for determination of internal amino acid sequence on an amino acid sequencer (PPSQ-10 manufactured by Shimadzu). It was revealed that the enzyme contains a peptide sequence consisting of

GlyPhePheGluAlaAspGluAsnAsnGluIleLys.

### Example 2

### Cloning of gene coding for FAO

Preparation of cDNA of N1-1 strain was conducted as follows. First, mRNA is extracted from the N1-1 strain. After the N1-1 strain was pre-cultured in YM medium (30°C, 5 ml, L-shaped tube), it was inoculated (initial concentration: 0.01, 30°C, 100 ml, 500 ml baffle) in an amount of 1% to M9 minimum medium (M9/FV) supplemented with fructosyl valine or FV (final concentration: 0.48%) as a sole nitrogen source. The cells were collected at about OD₆₆₀ = 3 (5,000 g, 15 min, 4°C), and resuspended in 200 µl of buffer A (1 M sorbitol, 100 mM EDTA and 14 mM mercaptoethanol). A zymolyase solution (20 µl) (1,000 U zymolyase/1 ml buffer A) was added and mixed, then allowed to stand at 30°C for 30 minutes. After centrifugation (15,000 rpm, 10 minutes, 4°C), the precipitate was suspended in 1 ml of Isogen and allowed to stand at room temperature for 5 minutes. Chloroform (0.2 ml) was added and mixed, and allowed to stand at room temperature for 3 minutes. After centrifugation (15,000 rpm, 15 minutes, 4°C), 0.5 ml of isopropanol was added to the aqueous phase and mixed, and allowed to stand at room temperature for 10 minutes. After centrifugation (15,000 rpm, 10 minutes, 4°C), 1 ml of 70% ethanol was added to the precipitate and mixed. After centrifugation (15,000 rpm, 5 minutes, 4°C), the precipitate was dried *in vacuo* for 15 minutes. cDNA was synthesized from the mRNA thus prepared.

A fragment containing FAO structural gene was amplified by a common method where cDNA is prepared by RT-PCR. First, mRNA was subjected to a reversed transcription reaction using an oligo dT adaptor primer according the to manual of RNA-PCR kit (AMV, Ver. 2.1, Takara). The cDNA was purified by precipitating with ethanol, and PCR was carried out using the cDNA. The PCR primers were FAO-F1 designed from a conserved sequence of already-reported FAODs and FAO-R2 designed from 13 amino acid residues obtained from amino acid sequence analysis of FAOD derived from N1-1 strain.
FAO-F1 5'-GGXACXTGGGGXWSXWSXACXGCXYTXCA-3'
FAO-R2

3'-TCYTCRTYXGGYTCVAWRAARAAXCC-5'

in which S = C + G, Y = C + T, R = A + G, X = A + C + G + T, W = A + T, V = A + C + G.

PCR amplification was carried out under the following reaction conditions:
one cycle of:
   at 94°C for 1 minute,
   at 60°C for 30 seconds and
   at 72°c for 30 seconds; and
25 cycles of:
   at 98°C for 30 seconds,
   at 60°C for 30 seconds,
   at 72°C for 7 minutes and
   at 72°C for 8 minutes.

TaKaRa LA *Taq*™ was used as a Taq polymerase. The PCR product was purified by glass milk purification according to the manual attached to Gene Clean II kit, then subcloned into a vector according to the manual attached to pGEM-T Vector System. A transformant carrying a vector having an insert was selected by color selection. The transformant was cultured in LB medium (37°C, 1.8 ml, test tube), then the cells were collected and plasmid was extracted. The plasmid was analyzed for the nucleotide sequence of the insert using M13 Primer Forward and M13 Primer Reverse (Takara Bio). Preparation and analysis of the sample were conducted according to the manual attached to ABI Prism 310 Genetic Analyzer (Perkin-Elmer Applied Biosystem).

Then PCR was carried out to analyze the nucleotide sequence of the C-terminal region. PCR was conducted using the purified cDNA as a template. The PCR primers were FAO-F3 designed from a sequence obtained by a nucleotide sequence analysis of the insert and a primer sequence of adapter primer.
FAO-F3: 5'-ATTTCAAAGTGACGGATGAAGAAGCTAAAG-3'
Adapter primer:

3'-CGCAGTTTTCCCAGTCACGAC-5'

On the basis of the internal partial sequence information of FAO determined from cDNA obtained as above, the full length structural gene of the enzyme was cloned by inverse PCR using a genomic DNA of N1-1 strain as a template.

Genomic DNA from N1-1 was extracted as follows. After N1-1 strain was cultured in YM medium (30°C, 100 ml, 300 ml baffle), 25 ml of ethanol and 1 mol of 0.5M EDTA were added to 25 ml of the culture medium and allowed to stand at -30°C for 30 minutes. The cells were collected (5.000G, 15 minutes, 4°C) and resuspended in 1 ml of aseptic ultrapure water. After centrifugation (8,000 rpm, 5 minutes, 4°C), the cells were resuspended in 0.5 ml of spheroplast buffer (1.2 M sorbitol, 0.1 M EDTA, 1% mercaptoethanol and 0.1% zymolyase) and allowed to stand at 30°C for 30 minutes. Proteinase K buffer (0.5 ml) (50 mM EDTA, 0.3% SDS and 0.01% proteinase) was added and mixed, and allowed to stand at 65°C for 30 minutes. 5M potassium acetate (0.2 ml) was added and mixed, and allowed to stand on ice for 10 minutes. After centrifugation (10,000 rpm, 15 minutes, 4°C), the supernatant liquid was precipitated with ethanol, and the precipitate was dried *in vacuo* for 15 minutes. The precipitate was dissolved in 500 µl of TE buffer (10 mM NaCl, 20 mM Tris-HCl (pH 8.0) and 1 mM EDTA), 5 µl of RNase was added and the mixture was allowed to stand at 37°C for 30 minutes. Then it was extracted with phenol-chloroform and precipitated with ethanol. The precipitate was dried *in vacuo* and dissolved in 1 ml of aseptic ultrapure water.

The genomic DNA derived from the N1-1 strain was then used for amplification of FAOD structural gene fragment by inverse PCR. The genomic DNA was digested with a restriction enzyme and allowed for self-ligation according to the manual attached to DNA Ligation Kit Ver. 2. PCR was conducted using the resulting cyclic DNA as a template. The primers FAO-F5 and FAO-R6 designed from the partial sequence of the structural gene of FAO were used.
FAO-F5 5'-GTGCATACGAAGAATGCAAACGATTGGGAGTGG-3'
FAO-R6

3'-CCATCCGTTATCTCCGTCGAGAACATATCCTC-5'

PCR amplification was carried out under the following conditions:
one cycle of:
   at 94°C for 1 minutes,
   at 60°C for 30 seconds and
   at 72°C for 30 seconds; and
25 cycles of:
   at 98°C for 30 seconds,
   at 60°C for 30 seconds,
   at 72°c for 7 minutes and
   at 72°C for 8 minutes.

TaKaRa LA *Taq*™ was used as a Taq polymerase. The PCR product was purified again and its nucleotide sequence was analyzed according to a common method. On the basis of FAO gene sequence information obtained above, a gene containing a full length fragment was prepared by PCR amplification from the N1-1 genomic DNA. In order to amplify the sequence containing the gene region, the following primers were designed.
FAO-NcoI: 5'-ATCACCATGGAGTCGATAATTATAGTTGG-3'
FAO-XbaI:

3'-TTGATTCTAGACATGTATGTTGTAATCTTG-5'

Using the primers, genomic DNA containing the fragment of interest was amplified by PCR.

The reaction was conducted according to the following cycles:
at 94°C for 5 minutes
(at 94°C for 30 seconds
at 55°C for 30 seconds
at 72°C for 1 minute) x 25 cycles
at 72°C for 5 minutes.

The nucleotide sequence of FAO gene obtained by the above-mentioned method was confirmed by a fully automated nucleotide sequence analyzer by a common method. The amino acid sequence of FAO was deduced from the nucleotide sequence determined as above.

### Example 3

### Construction of vector for recombinant production

FAO gene may be transferred from the vector obtained as above to a recombinant vector capable of replicating in a microorganism. For example, FAO gene is recovered from the vector with a restriction enzyme or PCR and then ligated to another vector fragment. Transformation of a microorganism with such a vector may be carried our by electroporation or a competent cell method by treatment with calcium.

FAO derived from N1-1 strain was recombinantly produced using *Escherichia coli.* Primers complementary to the N-terminal and C-terminal were designed from the structural gene sequence information of FAO derived from N1-1 strain. In designing the primers, *Nco* I recognition sequence (FAO-Nco I) was added to the primer of the N-terminal side while *Xba* I recognition sequence (FAO-Xba I) was added to the primer of the C-terminal side.
FAO-NcoI: 5'-ATCACCATGGAGTCGATAATTATAGTTGG-3'
FAO-XbaI:

3'-TTGATTCTAGACATGTATGTTGTAATCTTG-5'

FAOD structural gene was amplified by PCR using the above-mentioned primers using genomic DNA or cDNA was used as a template. The PCR product was digested with *Nco* I and *Xba* I and ligated (DNA Ligation Kit, Ver. 2 ) with a high-expression vector pTrc99a (Invitrogen) digested with the same restriction enzymes to obtain a plasmid (pTN1). A recombinant FAOD was produced with *Escherichia coli* transformed with the plasmid pTN1.

### Example 4

### Production of FAO by recombinant Escherichia coli

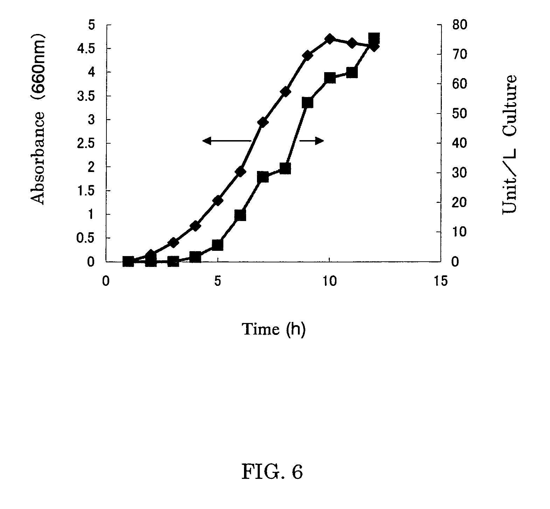

Production of FAO was conducted using *Escherichia coli* DH5α/pTNl transformed with a plasmid pTN1 containing structural gene of FAO. The transformant was inoculated to 3 ml of LB medium containing 50 µg/ml of ampicillin and cultured at 37°C for 12 hours. The cells were collected by centrifugation. The cells were disrupted by a French press (1,500 kgf) and a supernatant water-soluble fraction (10 mM potassium phosphate buffer, pH 6.0) was separated by ultracentrifugation (4°C, 160,400 × g, 90 minutes). The transformed *Escherichia coli* expressed FAO within the cells. When the enzyme is produced using wild-type *Pichia* sp N1-1, induction and production of the enzyme will require the presence of a fructosylamine compound in the medium, which is not commercially available and is expensive. On the other hand, according to the recombinant method of the present invention it is possible to induce the enzyme production by adding IPTG which is commonly used in a recombination technique for *Escherichia coli.* Thus, the method of the present invention is economically advantageous. In addition, the recombinant FAO was obtained in an amount of not less than 1.5-fold per unit protein as compared with the method using wild-type *Pichia* sp. N1-1 strain and fructosyl valine (Fig. 6).

### Example 5

### Purification of enzyme

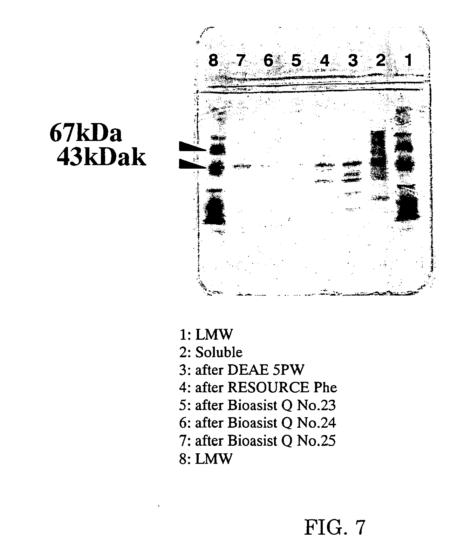

Purification of recombinant FAO was conducted as follows. First, a water-soluble fraction containing the enzyme was prepared from recombinant *Escherichia coli.* pTN1/DH5α was cultured in LB medium (37°C, 71, 101 fermenter, 50 µg/ml ampicillin), then induced with IPTG (final concentration: 0.3 mM) at about OD₆₆₀ = 0.7 and culture temperature was lowered to 30°C. The cells were suspended in 100 mM PPb (pH 7.0) and disrupted four times using a French press. The supernatant liquid was subjected to ultracentrifugation (40,000 g, 90 minutes) and the supernatant was dialyzed against 10 mM PPb (pH 7.0) at 4°C overnight to prepare a water-soluble fraction. The water-soluble fraction was further subjected to a liquid chromatography as follows to prepare a purified enzyme. The enzyme was further purified with an anion-exchange chromatography (DEAE-5PW). The water-soluble fraction was adsorbed to an anion-exchange chromatography column DEAE-5PW (5.0 mm I. D. x 5 cm, Tosoh) equilibrated with 10 mM PPb (pH 7.0). After equilibration with 10 mM PPb (pH 7.0) in a 3-fold amount of the column volume, FAOD was eluted with 10 mM PPb (pH 7.0) containing 0.7 M of NaCl. The flow rate was set to 1 ml/min and eluate was collected every one minute. Absorption wavelength of 280 nm was used to monitor eluate. The active fraction was separated using 35% ammonium sulfate and the supernatant liquid was subjected to hydrophobic chromatography. The active fraction was adsorbed to a hydrophobic chromatography column Resource Phe (1 ml, Pharmacia) equilibrated with 10 mM PPb (pH 6.5) containing 35% ammonium sulfate. After equilibration with 10 mM PPb (pH 6.5) containing 35% ammonium sulfate in a 3-fold amount of the column volume, FAOD was eluted with 10 mM PPb (pH 6.5) . The flow rate was set to 2 ml/min and eluate was collected every one minute. The active fraction was separated with 45% ammonium sulfate, then the precipitate was dissolved in 10 mM PPb (pH 7.0) containing 1% mannose and 100 µM FAD and dialyzed against the same buffer at 4°C for 6 hours. It was further dialyzed against 10 mM PPb (pH 8.0) containing 100 µM FAD at 4°C for 6 hours. The dialyzed sample was used for the next anion-exchange chromatography. The sample was adsorbed to an anion-exchange chromatography column Bioasit Q (4.6 mm. I. D. x 5 cm, Tosoh) equilibrated with 10 mM PPb (pH 8.0). After equilibration with 10 mM PPb (pH 8.0) in a 3-fold amount of the column volume, FAOD was eluted with 10 mM PPb (pH 7.0) containing 0.3M NaCl. The flow rate was set to 1 ml/min and eluate was collected every one minute. The active fraction was dialyzed against 10 mM PPb (pH 7.0) at 4°C overnight. Degree of purification of the sample was examined by SDS/PAGE. The sample was subjected to electrophoresis using Phast Gel 8-25, and the gel was stained with silver. Sample preparation, electrophoresis and staining was conducted in accordance with the manual attached to Phast System™. Electrophoretic picture of the purified enzyme is shown in Fig. 7.

### Example 6

### Determination of enzymatic activity

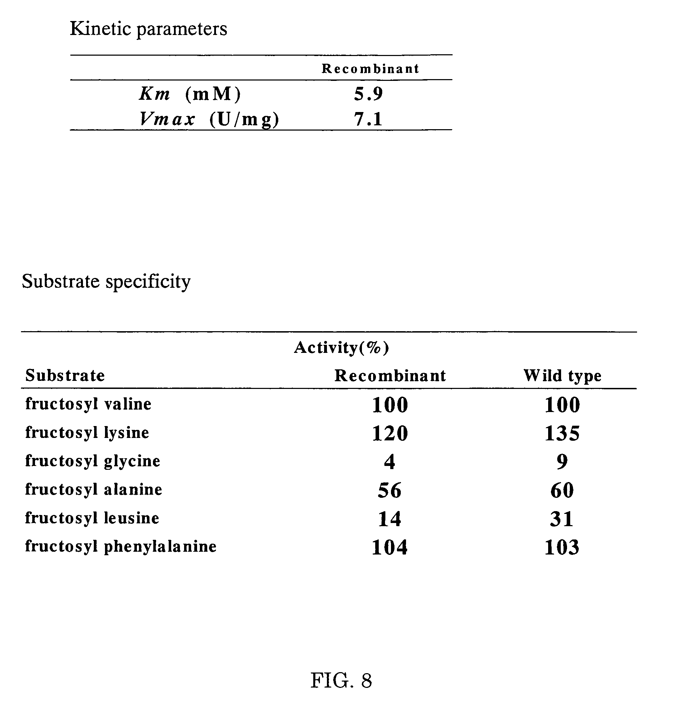

Reactivity of the purified enzyme prepared in Example 5 to fructosyl valine, N^{ε}-fructosyl lysine, fructosyl glycine, fructosyl alanine, fructosyl leucine, fructosyl phenylalanine, glucose and sarcosine was examined. The measurement was conducted using a solution containing a substrate (10 mM), PPb (pH 7.0, 50 mM), phenol (1.5 mM), 4-aminoantipyrine (1.5 mM) and peroxidase (2U/ml), and changes in the absorbance at 505 nm were measured according to the POD/Phenol/4A.A. method. Fig. 8 shows the activity and substrate specificity of the enzyme, and comparison of those of recombinant FAO produced by *Escherichia coli* with those of the enzyme produced by yeast *Pichia* sp. N1-1 strain of wild type. It was demonstrated that FAO having higher activity can be produced using a recombinant DNA technique.

### Example 7

### Assay of fructosylamines

Fructosyl-valine was assayed using the fructosylamine oxidase of the present invention. A measuring system using a peroxidase/4-aminoantipyrine system was employed to determine the dependency of fructosyl valine oxidizing activity of the fructosylamine oxidase (FAO) on the fructosyl valine concentration. The reaction was conducted at room temperature for 1 minute in the presence of 10 mM potassium phosphate buffer (pH 7.0) containing 1. 5 mM 4-aminoantipyrine, 2.0 mM phenol and 2U/ml peroxidase, and changes in the absorbance at 505 nm were monitored using a spectrophotometer. The enzymatic reaction rate was defined as a reducing rate of the absorbance. Linearity was noted between 0.1 mM to 5 mM and fructosyl valine was able to be quantitatively determined within that range.

### Example 8

### Enzyme sensor of fructosyl valine oxygen electrode type

FAO (100 microliters) (5.7 mg protein/ml) prepared according to Example 5 was impregnated to Kim Wipe, applied to an oxygen electrode manufactured by DKK and covered with a permeable membrane. This oxygen electrode was inserted into 10 ml of PBS (pH 7.4) kept at 30°C. A 1M aqueous solution (50 microliters) of fructosyl valine was appropriately added and decrease in electric current was observed. Fructosyl valine was able to be quantitatively determined within a range of 5 mM to 20 mM by the enzyme sensor using the novel fructosylamine oxidase of the present invention.

### Example 9

### Enzyme sensor of mediator type

FAO (corresponding to 0.34 unit) prepared according to Example 5 was freeze-dried, mixed with 20 mg of carbon paste and filled in a carbon paste electrode, and then the surface of the electrode was made flat on a filter paper. The surface was cross-linked with 1% glutaraldehyde for 30 minutes followed by treating with 10 mM lysine for 20 minutes to block the unreacted aldehyde groups. This enzyme electrode was equilibrated in a 20 mM potassium phosphate buffer (pH 7.0) by stirring at room temperature for 1 hour or more. A 20 mM potassium phosphate buffer (pH 7.0) containing 1 mM (as a final concentration) of methoxy-PMS as a mediator was used as a reaction solution in a total volume of 10 ml. The carbon paste electrode was used as a working electrode. Platinum electrode was used as a counter electrode and Ag/AgCl electrode was used as a reference electrode. The electrodes were inserted in the reaction solution and potential of +0.15V was applied. Measurement was conducted at 25°C. When the electric current value became steady (about 1 hour), a substrate (fructosyl valine) solution was injected by 100 µl portions. The difference between the peak electric current value observed upon injection of the substrate and the steady current value was defined as a response electric current value. The electric current value when no fructosyl valine was added was defined as 0 A and the response current value was calculated. The response current value was dependent on the concentration of fructosyl valine. Concentration of the injected fructosyl valine and the response current value showed linearity within a range of 0.1 mM to 0.6 mM, demonstrating that fructosyl valine can be measured within that range. After injection of the sample, it took about 4 minutes until a steady current value was achieved.

### Example 10

### Sensor of hydrogen peroxide detection type

First, enzyme-fixed membrane was prepared. FAO (50 µl) (corresponding to 0.05 unit) prepared according to Example 5 and 0.23 g of an aqueous solution of PVA-SbQ which is an optically cross-linking prepolymer were mixed and thinly spread on a plate to the area of 21 cm². After it was air-dried in a dark place, both sides thereof were exposed to fluorescent light for 5 minutes each. The membrane to be used as an optically immobilized enzyme membrane was stored at 4°C. The membrane was placed on the surface of a platinum electrode (BAS; Model No. 11-1012), covered with a Nylon net and fixed with an O ring to prepare an enzyme electrode, which was used as a working electrode. Ag/AgCl was used for a reference electrode and platinum electrode was used as a counter electrode. To a constant-temperature cell (25°C) was added 10 ml of 500 mM potassium phosphate buffer (pH 7.0) and the optically cross-linking resin immobilized enzyme electrode was inserted. Potential of +0.6V vs. Ag/AgCl was applied using a potentiostat. After the electric current became steady (about 1 hour), 100 µl portions of the substrate (fructosyl valine) were injected. The difference between the steady electric current value and the peak current value observed upon injection of the substrate was defined as a response current value. Using the batch-type system constructed in this example, a response curve depending upon the concentration of fructosyl valine was obtained. Linearity was noted within a range of the injected fructosyl valine concentration of from 0.05 mM to 2 mM, demonstrating that fructosyl valine can be measured within that range.

### Example 11

### Prussian Blue type sensor

On a glassy carbon electrode (BAS, Model No. 11-2012), 2mM (final concentration) potassium hexacyano iron (III) and 2 mM of iron (III) chloride were mixed with 0.1 M potassium chloride buffer, and potential of +0. 4 V vs Ag/AgCl was applied at 25°C for 60 seconds to adhere a film. Then potentials of -0.05 V to 0.35 V were applied for ten times to form a film to obtain an electrode fixed with Prussian Blue film. The Prussian Blue film-fixed electrode was covered with the FAO-immobilized membrane prepared in Example 5 (0.136 unit). Ag/AgCl was used as a reference electrode while platinum electrode was used as a counter electrode. A 500 mM potassium phosphate buffer (pH 7.0) (10 ml) was added to a constant-temperature cell (25°C). To the Prussian Blue film-fixed enzyme electrode was applied 0.05 V vs. Ag/AgCl with a potentiostat. After the current value became steady (about 1 hour), 100 µl portions of a substrate (fructosyl valine) solution were injected. The difference between the steady electric current value and the peak current value observed upon injection of the substrate was defined as a response current value. The batch-type system constructed in this example was able to provide reproducible results depending upon the concentration of fructosyl valine. Depending upon the injected fructosyl valine concentration, linearity was noted within a range of 0.1 mM to 1.6 mM, demonstrating that fructosyl valine can be measured within that range. After injection of the sample, it took about 6 minutes until a steady current value was achieved.

### Industrial Applicability

In accordance with the present invention, it has now been succeeded in cloning of structural gene of fructosylamine oxidase from *Pichia* sp., which is used as an enzyme for the measurement of glycated hemoglobin (HbAlc). The enzyme may be recombinantly produced in *Escherichia coli* in large quantities. The enzyme may be applied for analysis of clinical samples.

## Claims

1. A protein having a fructosylamine oxidase activity of the following (a) or (b):
(a) a protein comprising the amino acid sequence as set forth in SEQ ID NO: 1 of the Sequence Listing;
(b) a protein comprising an amino acid sequence where one or several amino acid residue(s) is/are deleted, substituted or added in the amino acid sequence of (a) and having a fructosylamine oxidase activity.

2. A fructosylamine oxidase containing the sequence

GlyPhePhePheGluAlaAspGluAsnAsnGluIleLys.

3. A fructosylamine oxidase having any of the sequences of the following (e) to (h):

(e) PheHisTyrAspTyrValAlaProLeuAlaLysProAsnSerLysGluArg;

(f)

AspAlaProLeuLeuHisAspLysGluTyrTyrGluGluLeuGlnLysAsnGlyLeuA

rgAsnTyrArgTyrIleSerThr;

(g) ThrLysGlyAspLysGlyLeuAspProGluAspLys; and

(h) TrpValSerValGluAsnProThrProHisLysLeuGlu.

4. The fructosylamine oxidase according to any one of claims 1 to 3, which is derived from *Pichia* sp. N1-1 strain or from genus *Pichia.*

5. A gene coding for a fructosylamine oxidase which is a protein of the following (a) or (b):
(a) a protein comprising the amino acid sequence as set forth in SEQ ID NO: 1 of the Sequence Listing;
(b) a protein comprising an amino acid sequence where one or several amino acid residue(s) is/are deleted, substituted or added in the amino acid sequence of (a) and having a fructosylamine oxidase activity.

6. A gene coding for a fructosylamine oxidase which is any one of the following (c) or (d):
(c) DNA comprising the nucleotide sequence as set forth in SEQ ID NO: 2 of the Sequence Listing;
(d) DNA where one or several nucleotide(s) is/are deleted, substituted or added in the above sequence (c) and codes for a protein having a fructosylamine oxidase activity.

7. A recombinant vector containing the gene as claimed in claim 5 or 6.

8. A transformant or a transfectant transformed with the recombinant vector as claimed in claim 7.

9. A process for the production of a fructosylamine oxidase comprising culturing the transformant as claimed in claim 8, and collecting the fructosylamine oxidase from the culture.

10. A fructosylamine oxidase produced by the process as claimed in claim 9.

11. A spectroscopic analysis method for a fructosylamine compound using the fructosylamine oxidase as claimed in any one of claims 1 to 4 or 10.

12. An electrochemical analysis method for a fructosylamine compound using the fructosylamine oxidase as claimed in any one of claims 1 to 4 or 10.

13. An electrochemical analysis method for fructosyl valine using the fructosylamine oxidase as claimed in any one of claims 1 to 4 or 10.

14. A method for the assay of HbA1c comprising digesting HbA1c in a sample to generate fructosyl valine, and analyzing the fructosyl valine spectroscopically using the fructosylamine oxidase as claimed in any one of claims 1 to 4 or 10.

15. A method for the assay of fructosamine comprising digesting fructosamine in a sample to generate a fructosylamine compound, and analyzing the fructosylamine compound spectroscopically using the fructosylamine oxidase as claimed in any one of claims 1 to 4 or 10.

16. A method for the assay of glycated albumin comprising digesting glycated albumin in a sample to generate a fructosylamine compound, and analyzing the fructosylamine compound spectroscopically using the fructosylamine oxidase as claimed in any one of claims 1 to 4 or 10.

17. An electrochemical analysis method for HbA1c using the fructosylamine oxidase as claimed in any one of claims 1 to 4 or 10.

18. An electrochemical analysis method for fructosamine using the fructosylamine oxidase as claimed in any one of claims 1 to 4 or 10.

19. An electrochemical analysis method for glycated albumin using the fructosylamine oxidase as claimed in any one of claims 1 to 4 or 10.

20. A kit for assay of fructosyl valine comprising the fructosylamine oxidase as claimed in any one of claims 1 to 4 or 10.

21. A kit for assay of HbA1c containing the fructosylamine oxidase as claimed in any one of claims 1 to 4 or 10.

22. A kit for assay of fructosamine comprising the fructosylamine oxidase as claimed in any one of claims 1 to 4 or 10.

23. A kit for assay of glycated albumin comprising the fructosylamine oxidase as claimed in any one of claims 1 to 4 or 10.

24. An enzyme electrode having the fructosylamine oxidase as claimed in any one of claims 1 to 4 or 10 immobilized thereon.

25. An enzyme sensor comprising the enzyme electrode as claimed in claim 24 as a working electrode.
